# EUROPEAN PATENT APPLICATION

(11) **EP 3 586 765 A1**
(43) Date of publication of application: **01.01.2020**
(21) Application number: 19181509.1
(22) Date of filing: 20.06.2019
(51) Int. Cl.: A61B 17/04, A61B 17/062

(54) **POWER ENDO STITCH**

(30) Priority: 21.06.2018 US 201862687836 P; 16.05.2019 US 201916413765
(71) Applicant: Covidien LP, Mansfield, MA 02048 (US)
(72) Inventor: NICHOLAS, David, Trumbull, Connecticut 06611 (US); PRIBANIC, Russell, Roxbury, Connecticut 06783 (US); MARCZYK, Stanislaw, Stratford, Connecticut 06614 (US)
(74) Representative: Maschio, Antonio

(57) **Abstract**

An endoscopic stitching device includes a handle assembly and an elongate shaft assembly. The handle assembly includes an actuation assembly and a processor. The actuation assembly includes first, second, and third motors. The processor is electrically connected to the first, second, and third motors to control actuation of the first, second, and third motors. The elongate shaft assembly includes a main rod, first and second blade drive members, and a tool assembly. The main rod is operatively coupled with the first motor of the actuation assembly such that actuation of the first motor causes axial displacement of the main rod. The first and second blade drive members are operatively coupled with the second and third motors, respectively, such that actuation of the second and third motors causes axial displacement of the first and second blade drive members.

## Description

### CROSS-REFERENCE TO RELATED APPLICATIONS

This application claims the benefit of and priority to U.S. Provisional Patent Application Serial No. 62/687,836 filed June 21, 2018, the entire disclosure of which is incorporated by reference herein.

### BACKGROUND

### Technical Field

The present disclosure relates to devices for suturing or stitching and, more particularly, to powered devices for endoscopic suturing and/or stitching through an access tube or the like.

### Background

One of the advances in recent years to reduce the invasiveness of surgical procedures is endoscopic surgery. Generally, endoscopic surgery involves incising through body walls. Typically, trocars are utilized for creating the incisions through which the endoscopic surgery is performed. Trocar tubes or cannula devices are extended into and left in place in the abdominal wall to provide access for endoscopic surgical tools. A camera or endoscope is inserted through a relatively large diameter trocar tube which is generally located at the naval incision, and permits the visual inspection and magnification of the body cavity. The surgeon can then perform diagnostic and therapeutic procedures at the surgical site with the aid of specialized instrumentation, such as, forceps, cutters, applicators, and the like which are designed to fit through additional cannulas.

In many surgical procedures, including those involved in endoscopic surgery, it is often necessary to suture bodily organs or tissue. Suturing may be challenging during endoscopic surgery because of the small openings through which the suturing of bodily organs or tissues must be accomplished. Accordingly, a need exists for simple and effective devices for endoscopic suturing or stitching.

### SUMMARY

The present disclosure describes a device for suturing and stitching that demonstrates a practical approach to meeting the performance requirements and overcoming usability challenges associated with endoscopic suturing or stitching. In accordance with an embodiment of the present disclosure, there is provided an endoscopic stitching device including a handle assembly and an elongate shaft assembly.

The handle assembly includes an actuation assembly and a processor. The actuation assembly includes first, second, and third motors. The processor is electrically connected to the first, second, and third motors to control actuation of the first, second, and third motors.

The elongate shaft assembly includes a main rod, first and second blade drive members, and a tool assembly. The main rod is operatively coupled with the first motor of the actuation assembly such that actuation of the first motor causes axial displacement of the main rod. The first and second blade drive members are operatively coupled with the second and third motors, respectively, such that actuation of the second and third motors causes axial displacement of the first and second blade drive members. The tool assembly includes first and second jaws and first and second blades. The first and second jaws are operatively coupled with the main rod of the elongate shaft assembly such that axial displacement of the main rod transitions the first and second jaws between open and closed positions. The first and second blades are slidably disposed in the respective first and second jaws. Each of the first and second blades is configured to engage a needle received in the first or second jaws. The first and second blades are operatively coupled with the first and second blade drive members, respectively, such that actuation of the second and third motors causes axial displacement of the first and second blades.

In an embodiment, the handle assembly may further include a first actuation switch configured to actuate the first motor to impart axial displacement to the main rod, which in turn, transitions the first and second jaws between the open and closed positions.

In another embodiment, the handle assembly may further include a potentiometer operatively coupled to the first actuation switch to enable proportional control of the jaws.

In yet another embodiment, the first actuation switch may further include a gear assembly operatively coupled with the potentiometer to improve sensor resolution.

In still yet another embodiment, the handle assembly may further include a first lead screw coupled with an output shaft of the first motor for concomitant rotation therewith, and a first coupling nut threadably coupled with the first lead screw and securely fixed with the main rod such that actuation of the first motor causes axial displacement of the main rod.

In still yet another embodiment, the handle assembly may further include second and third lead screws coupled with respective output shafts of the second and third motors for concomitant rotation therewith, and second and third coupling nuts threadably coupled with the respective second and third lead screws and securely fixed with the respective first and second blade drive members such that actuation of the second and third motors causes axial displacement of the respective first and second blades.

In still yet another embodiment, the handle assembly may further include second and third guide blocks configured to slidably receive a least a portion of the respective second and third coupling nuts thereon, while inhibiting rotation of the second and third coupling nuts about the respective second and third lead screws.

In still yet another embodiment, the handle assembly may further include a second actuation switch configured to actuate the second and third motors in order to cause reciprocating axial displacement of the first and second blade drive members in opposite directions.

In an embodiment, the handle assembly may further include a battery pack electrically coupled to the actuation assembly and the processor to supply power thereto.

In another embodiment, the batter pack may be removably attached to a housing of the handle assembly.

In yet another embodiment, the handle assembly may further include a third actuation switch operatively coupled with the processor and the second and third motors such that actuation of the third actuation switch causes retraction of the first and second blades.

In an embodiment, actuation of the third actuation switch may cause axial displacement of the first and second blades in a proximal direction.

In another embodiment, proximal displacement of the main rod may transition the first and second jaws to the closed position.

In accordance with another embodiment of the present disclosure, there is provided a powered handle assembly for use with a stitching device including an actuation assembly, a processor, a first actuation switch, and a second actuation switch. The actuation assembly includes first, second, and third motors. The processor is electrically connected to the first, second, and third motors to control actuation of the first, second, and third motors. The first actuation switch is electrically connected to the processor to control actuation of the first motor operatively coupled with a main rod of the stitching device. The second actuation switch is electrically connected to the processor to control actuation of the second and third motors coupled with respective first and second blade drive members of the stitching device. Actuation of the first actuation switch causes axial displacement of the main rod of the stitching device, and actuation of the second actuation switch causes actuation of the second and third motors, which, in turn, causes axial displacement of the first and second blade drive members in opposite directions.

### DETAILED DESCRIPTION OF THE DRAWINGS

The foregoing objects, features and advantages of the disclosure will become more apparent from a reading of the following description in connection with the accompanying drawings, in which:
FIG. 1 is a perspective view of a handle assembly for use with a stitching device in accordance with an embodiment of the present disclosure;
FIG. 2 is a perspective view of an elongate shaft assembly of the stitching device;
FIG. 3 is a top view of a tool assembly of the elongate shaft assembly of FIG. 2;
FIG. 4 is a perspective view, with parts separated, of the elongate shaft assembly of FIG. 2;
FIG. 5 is a partial, longitudinal cross-sectional view of the tool assembly of FIG. 2;
FIG. 6 is a perspective view of the handle assembly of FIG. 1 with a first half of a housing of the handle assembly removed;
FIG. 7 is a partial perspective view of the handle assembly of FIG. 1 with a second half of the housing and a printed circuit board of the handle assembly removed; and
FIG. 8 is a perspective view of the handle assembly of FIG. 8 illustrating the printed circuit board.

### DETAILED DESCRIPTION

Embodiments of the present disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the present disclosure in unnecessary detail.

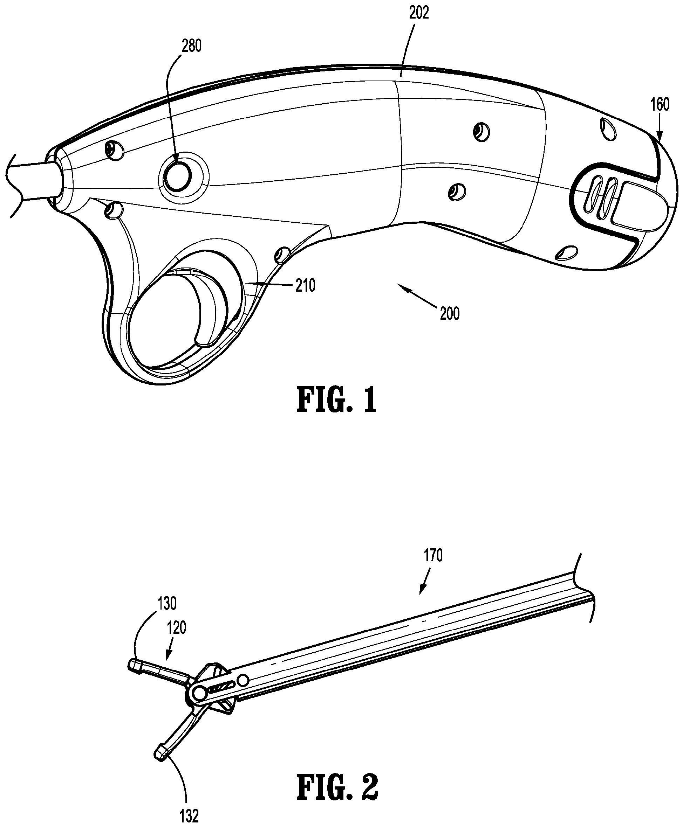

With reference to FIGS. 1 and 2, a handle assembly for use with a stitching device in accordance with an embodiment of the present disclosure is shown generally as a handle assembly 200. The stitching device may be adapted to be particularly useful in endoscopic or laparoscopic procedures, wherein an endoscopic portion of the stitching device such as, e.g., a tool assembly 120, is insertable into an operative site, via a cannula assembly or the like (not shown). The stitching device may include the handle assembly 200 and an elongate shaft assembly 170 extending distally from the handle assembly 200.

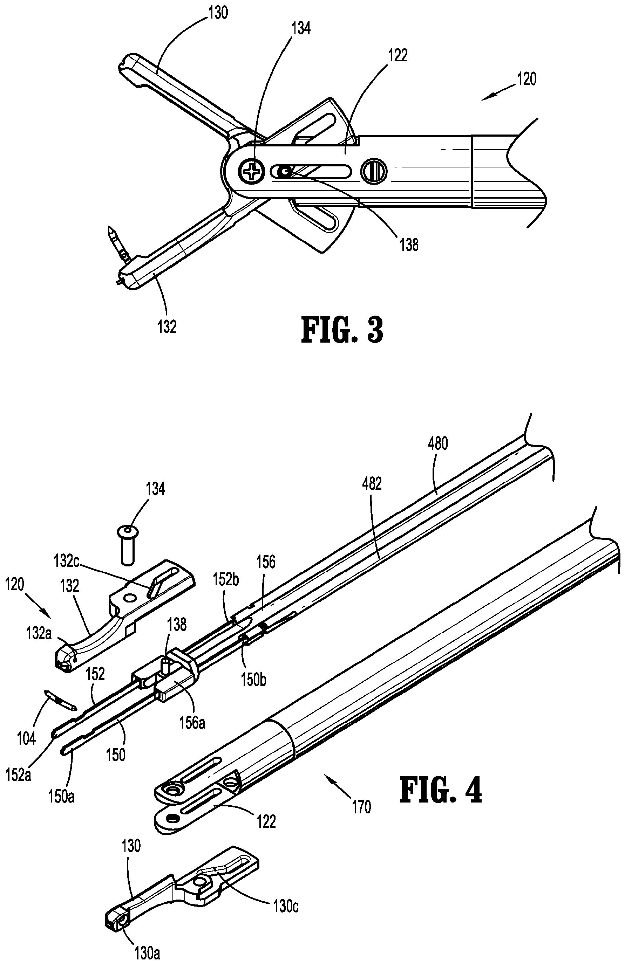

With reference now to FIGS. 3 and 4, the elongate shaft assembly 170 includes the tool assembly 120. The tool assembly 120 includes a support member 122 and jaws 130, 132 pivotably mounted on the support member 122 by means of a jaw pivot pin 134. To move the jaws 130, 132 between an open position and a closed position, the main rod 156 is operatively coupled to the jaws 130, 132. In particular, the main rod 156 has a camming pin 138 mounted at a distal end 156a thereof. The camming pin 138 rides in angled camming slots 130c, 132c defined in the respective jaws 130, 132 such that axial or longitudinal movement of the main rod 156 causes the jaws 130, 132 to be cammed between open and closed positions. The main rod 156 may be provided with, e.g., biasing members in the form of a return spring, to bias the main rod 156 toward an initial position, in which, e.g., the jaws 130, 132 are in the open position.

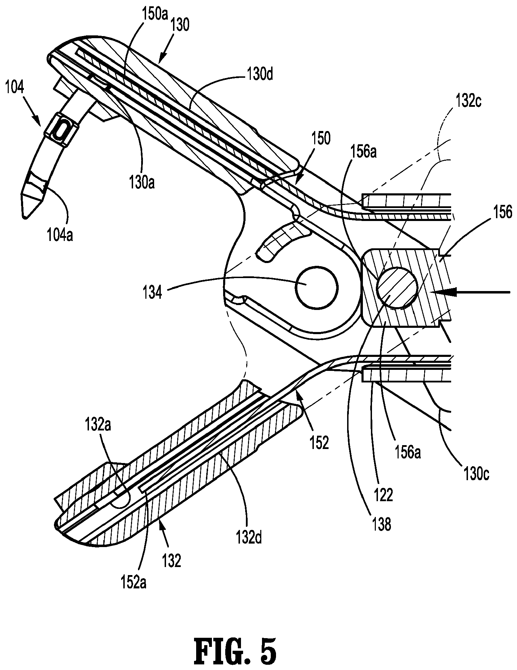

With reference to FIGS. 4 and 5, the tool assembly 120 further includes a pair of needle engaging members or blades 150, 152 which are slidably supported within the support member 122. Each blade 150, 152 includes a distal end 150a or 152a slidably extending into a blade receiving channel 130d or 132d of the corresponding jaw 130 or 132, and a proximal end 150b or 152b operatively coupled to a corresponding first or second blade drive member 480 or 482 extending through the elongate shaft assembly 170 and operatively coupled to the handle assembly 200. The first and second blade drive members 480, 482 are coupled with the respective blades 150, 152, such that reciprocating axial displacement of the first and second blade drive members 480, 482 provides reciprocating axial displacement of the blades 150, 152, enabling swapping of a needle 104 between the jaws 130, 132.

With particular reference to FIG. 5, the blade receiving channels 130d, 132d are dimensioned to at least partially intersect needle recesses 130a, 132a. Thus, by advancing the blade 150 or 152 within the corresponding blade receiving channel 130d or 132d, the distal end 150a or 152a of the corresponding blade 150 or 152 engages or "locks in" a groove 104a formed in the needle 104 when at least a portion of the needle 104 is received within the corresponding recess 130a or 132a. A suture (not shown) may be secured to the needle 104. The suture may include a plurality of barbs oriented to resist movement in a direction opposite to the direction of travel. Reference may be made to U.S. Patent No. 8,628,545, entitled "Endoscopic Stitching Devices," the entire contents of which are incorporated herein by reference, for a detailed description of the construction and operation of a tool assembly.

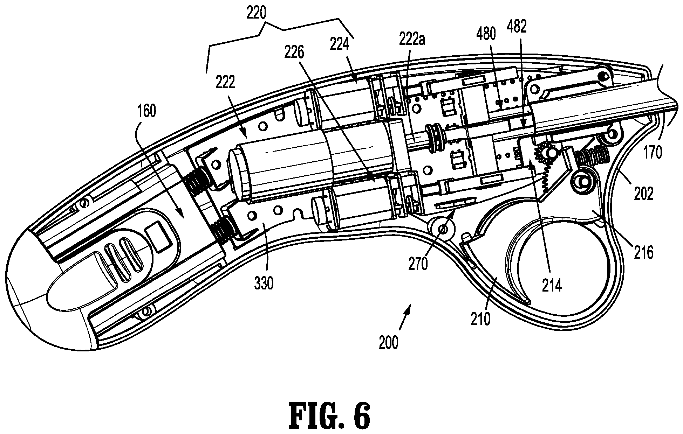

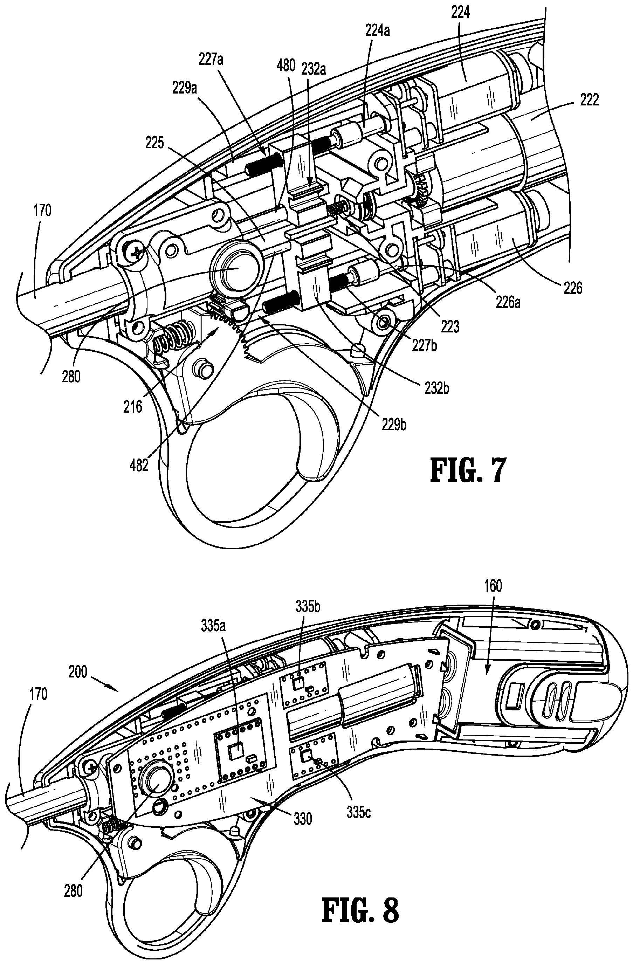

With reference now to FIGS. 6 and 7, the handle assembly 200 includes a housing 202 configured to receive an actuation assembly 220, a printed circuit board 330 including processors 335a, 335b, 335c (FIG. 8) to control the actuation assembly 220, and a battery pack 160 removably attached to the housing 202 and electrically connected to the actuation assembly 220 and the printed circuit board 330 to supply power thereto. The housing 202 includes an ergonomic structure providing comfort, ease of use, and intuitiveness such that when the housing 202 is gripped by a clinician, an index finger may be positioned to trigger a first actuation switch 210.

The first actuation switch 210 is operatively coupled to the main rod 156 (FIG. 5) to transition the jaws 130, 132 (FIG. 5) between the open and closed positions. In particular, the first actuation switch 210 is electrically coupled with a first motor 222 of the actuation assembly 220 such that when the first actuation switch 210 is triggered by, e.g., an index finger of the clinician, the first motor 222 is actuated and retracts the main rod 156, which, in turn, transitions the jaws 130, 132 to the closed position. Specifically, an output shaft 222a of the first motor 222 is coupled with a first lead screw 223 (FIG. 7) for concomitant rotation therewith. The first lead screw 223 is threadably coupled with a first coupling nut 225 (FIG. 7) that is securely coupled with the main rod 156 (FIG. 5) for concomitant axial displacement therewith. Under such a configuration, when the first lead screw 223 is rotated, the first lead screw 223 threadably engages the first coupling nut 225, which, in turn, causes axial displacement of the first coupling nut 225. In this manner, the main rod 156 may be axially displaced in order to transition the jaws 130, 132 between the open and closed positions.

The first actuation switch 210 may be coupled with, e.g., a potentiometer 214 (FIG. 6) or a similar rotary sensor, to enable proportional control of the jaws 130, 132 based on a position of the first actuation switch 210 when triggered by the clinician, e.g., the amount of jaw movement may correspond to the amount of depression of the first actuation switch 210. For example, the first actuation switch 210 depressed about half way would close the jaws 130, 132 about half way between the open and closed positions. Furthermore, the first actuation switch 210 may further include a gear assembly 216 (FIG. 7), in which, a gear ratio may be selectively chosen to improve, e.g., sensor resolution. In the absence of a detent mechanism, the jaws 130, 132 may transition to the open position when the first actuation switch 210 is released prior to reaching the closed position, at which time the needle 104 remains in the same jaw 130 or 132 prior to the reversal process. However, the handle assembly 200 may include a detent mechanism (not shown), which inhibits the jaws 130, 132 from transitioning back to the open position when the first actuation switch 210 is released prior to being fully squeezed, e.g., prior to the jaws 130, 132 reaching in the fully closed position.

With continued reference to FIGS. 6 and 7, the actuation assembly 220 further includes second and third motors 224, 226 operatively coupled with the first and second drive members 480, 482, respectively. In particular, output shafts 224a, 226a of the respective second and third motors 224, 226 are coupled with respective second and third lead screws 227a, 227b for concomitant rotation therewith. The second and third lead screws 227a, 227b threadably engage respective second and third coupling nuts 232a, 232b. The second and third coupling nuts 232a, 232b operatively connect the second and third lead screws 227a, 227b with the respective first and second drive members 480, 482. The second and third coupling nuts 232a, 232b are securely fixed with the respective first and second drive members 480, 482 for concomitant axial displacement. In particular, the second and third coupling nuts 232a, 232b are slidably disposed on respective first and second guide blocks 229a, 229b. Under such a configuration, when the second or third motor 224 or 226 is actuated, the corresponding second or third lead screw 227a or 227b rotates, which in turn, causes axial displacement of the second or third coupling nut 232a or 232b therealong. Axial displacement of the second or third coupling nut 232a or 232b causes concomitant axial displacement of the corresponding first or second drive member 480 or 482, which imparts axial displacement to the corresponding blade 150, 152 in the tool assembly 120. For example, the second or third coupling nuts 232a, 232b may include flags for optical position sensors.

With brief reference to FIG. 8, the processors 335a, 335b, 335c may control actuation of the respective first, second, and third motors 222, 224, 226. The printed circuit board 330 may further include a needle transfer switch 270 (FIG. 6). The needle transfer switch 270 may be configured to switch the needle 104 (FIG. 5) between the jaws 130, 132 (FIG. 5). Specifically, the needle transfer switch 270 may be pressed when the jaws 130, 132 are in the closed position in order to displace the blades 150, 152 in opposite directions, as will be discussed hereinbelow. For example, an increased force and detent, may provide tactile feedback to the clinician. Alternatively, a rotary switch position sensor may be used.

With reference back to FIG. 7, the printed circuit board 330 (FIG. 8) may further include a needle reload switch 280 that serves to retract the blades 150, 152 (FIG. 5) to release the needle 104. In this manner, the clinician may discard the used needle 104 from the tool assembly 120 (FIG. 5). Thereafter, a needle reload (not shown) including a new needle 104 may be attached to the tool assembly 120. Once the needle reload is attached, the jaws 130, 132 may be closed by triggering the first actuation switch 210, which, in turn, causes one of the jaws 130, 132 to engage the needle 104. At this time, the needle reload may be removed and the stitching device is ready to begin suturing. It is contemplated that the needle reload switch 280 may illuminate to indicate the status of the stitching device. For example, when the stitching device is in a suturing mode, e.g., the needle 104 is engaged with at least one of the jaws 130 or 132, the needle reload switch 280 may illuminate, and when the stitching device is in the reload mode, e.g., the needle 104 is disengaged from the jaws 130, 132, the needle reload switch 280 may be dimmed.

The stitching device is transitionable between the suture mode and the reload mode. In the suture mode, the jaws 130, 132 are in the open position and the needle 104 is loaded and held in one jaw 130 or 132. The jaws 130, 132 may be positioned about or over a target tissue and the first actuation switch 210 may be actuated to approximate the jaws 130, 132. As the jaws 130, 132 are approximated, the exposed end of the needle 104 is penetrated through the target tissue and enters opposed jaw 130 or 132. In particular, in order close the jaws 130, 132, the first actuation switch 210 of the handle assembly 200 is squeezed by the clinician, which, in turn, actuates the first motor 222 and causes axial displacement of the first coupling nut 225 coupled with the main rod 156. Proximal axial displacement of the main rod 156 transitions the jaws 130, 132 from the open position to the closed position.

At this time, in order to swap the needle 104 between the jaws 130, 132 in order to perform suturing, the needle transfer switch 270 (FIG. 6) is pressed by the clinician while the jaws 130, 132 are fully closed. When the needle transfer switch 270 is pressed, the second and third motors 224, 246 are actuated in order to cause reciprocating axial displacement of the first and second blade drive members 480, 482 in opposite directions. In this manner, the blades 150, 152 (FIG. 5) may be displaced in opposite directions to enable swapping of the needle 104 between the jaws 130, 132. As a result, the first and second blade drive members 480, 482 are axially displaced in opposite directions, which, in turn, causes reciprocating axial displacement of the blades 150, 152 of the tool assembly 120. In so doing, the needle 104 is swapped from one blade 150 or 152 to the other blade 150 or 152 when the first actuation switch 210 is released, whereby the needle 104 is loaded or held in the other jaw 130 or 132. Thus, closing of the jaws 130, 132 is affected by squeezing the trigger 210 and reciprocating axial displacement of the first and second blade drive members 480, 482 is done by pressing the needle transfer switch 270. Under such a configuration, the handle assembly 200 may be held in a stable manner since a light touch from an index finger is the only input required to operate the stitching device. In this manner, the handle assembly 200 eliminates the need for a manually operated toggle mechanism typically found in stitching devices for performing reciprocating axial displacement of the first and second blade drive members 480, 482, as well a pair of handles configured to be squeezed to close the jaws 130, 132.

In the reload mode, a loading or unloading of the needle 104 into or from one of the jaws 130, 132 may be performed. Specifically, the clinician may press the needle reload switch 280 (FIG. 7), which retracts both blades 150, 152 such that notches formed in respective blades 150, 152 are aligned with or in registration with the respective needle recesses 130a, 132a (FIG. 5) defined in the respective jaws 130, 132. With the notches of the blades 150, 152 aligned with or in registration with the respective needle recesses 130a, 132a, the needle 104 (FIG. 2) may be loaded into a selected one needle recess 130a, 132a of the jaws 130, 132 or unloaded from the needle recesses 130a, 132 of the jaws 130, 132.

In use, the stitching device is initially transitioned to the reload mode by pressing the needle reload switch 280. In this manner, the first and second blade drive members 480, 482 are displaced such that notches formed in respective blades 150, 152 are aligned with or in registration with the respective needle recesses 130a, 132a (FIG. 5) defined in the respective jaws 130, 132. With the notches of blades 150, 152 aligned with or in registration with the respective needle recesses 130a, 132a (FIG. 5) of the respective jaws 130, 132, the needle 104 (FIG. 2) may be positioned or loaded into a selected one needle recess 130a, 132a of the jaws 130, 132.

Once the needle 104 is loaded into one of the needle recesses 130a, 132a (FIG. 5) of the jaws 130, 132, the first actuation switch 210 is squeezed so that only one blade 150, 152, is in engagement with the needle 104 (FIG. 5), and the other blade 150, 152 is disengaged from the needle 104. With the jaws 130, 132 in the open position and the needle 104 loaded and held in one jaw 130 or 132, the jaws 130, 132 may be positioned about or over a target tissue. In order to close jaws 130, 132 and swap the needle 104 between the jaws 130, 132, the first actuation switch 210 is squeezed to fully close the jaws 130, 132, and thereafter the needle transfer switch 270 is pressed.

As the jaws 130, 132 are approximated, the exposed end of the needle 104 is penetrated through the target tissue and enters opposed jaw 130 or 132. With the needle 104 in opposed jaw 130 or 132, the needle transfer switch 270 is pressed causing the first and second blade drive members 480, 482 to be axially displaced in opposite directions, which, in turn, causes reciprocating axial displacement of the blades 150, 152 (FIG. 5) of the tool assembly 120. In so doing, the needle 104 is swapped from one blade 150 or 152 to the other blade 150 or 152, and thus, loaded or held in the other jaw 130 or 132. With the needle 104 being swapped from one blade 150, 152 to the other blade 150, 152, the first actuation switch 210 may be released to thereby open the jaws 130, 132 and draw the needle 104 through the target tissue. In so doing, the suture is also drawn through the tissue. The process is repeated, passing the needle 104 between the jaws 130, 132 and drawing the suture through the target tissue, thereby suturing the target tissue as needed or desired.

The needle 104 may be unloaded from the jaws 130, 132 in order to be replaced with a new needle 104 during or after the surgical procedure. In order to replace the needle 104, the needle reload switch 280 is pressed, which causes the notches formed in the respective blades 150, 152 to be aligned with or in registration with the respective needle recesses 130a, 132a (FIG. 5) defined in the respective jaws 130, 132. With the notches of the blades 150, 152 aligned with or in registration with the respective needle recesses 130a, 132a, the needle 104 may be removed from the needle recesses 130a, 132a of the jaws 130, 132 and a new needle 104 may be loaded into one of the jaws 130, 132.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure.

Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:-
1. An endoscopic stitching device comprising:
   a handle assembly including:
      an actuation assembly including first, second, and third motors; and
      a processor electrically connected to the first, second, and third motors, the processor configured to control actuation of the first, second, and third motors; and
   an elongate shaft assembly including:
      a main rod operatively coupled with the first motor of the actuation assembly such that actuation of the first motor causes axial displacement of the main rod;
      first and second blade drive members operatively coupled with the second and third motors, respectively, such that actuation of the second and third motors causes axial displacement of the first and second blade drive members; and
      a tool assembly including:
         first and second jaws operatively coupled with the main rod of the elongate shaft assembly such that axial displacement of the main rod transitions the first and second jaws between open and closed positions; and
         first and second blades slidably disposed in the respective first and second jaws, each of the first and second blades configured to engage a needle received in the first or second jaws, the first and second blades operatively coupled with the first and second blade drive members, respectively, such that actuation of the second and third motors causes axial displacement of the first and second blades.
2. The endoscopic stitching device according to paragraph 1, wherein the handle assembly further includes a first actuation switch configured to actuate the first motor to impart axial displacement to the main rod, which in turn, transitions the first and second jaws between the open and closed positions.
3. The endoscopic stitching device according to paragraph 2, wherein the handle assembly further includes a potentiometer operatively coupled to the first actuation switch to enable proportional control of the jaws.
4. The endoscopic stitching device according to paragraph 3, wherein the first actuation switch further includes a gear assembly operatively coupled with the potentiometer to improve sensor resolution.
5. The endoscopic stitching device according to paragraph 1, wherein the handle assembly further includes a first lead screw coupled with an output shaft of the first motor for concomitant rotation therewith, and a first coupling nut threadably coupled with the first lead screw and securely fixed with the main rod such that actuation of the first motor causes axial displacement of the main rod.
6. The endoscopic stitching device according to paragraph 1, wherein the handle assembly further includes second and third lead screws coupled with respective output shafts of the second and third motors for concomitant rotation therewith, and second and third coupling nuts threadably coupled with the respective second and third lead screws and securely fixed with the respective first and second blade drive members such that actuation of the second and third motors causes axial displacement of the respective first and second blades.
7. The endoscopic stitching device according to paragraph 6, wherein the handle assembly further includes second and third guide blocks configured to slidably receive a least a portion of the respective second and third coupling nuts thereon, while inhibiting rotation of the second and third coupling nuts about the respective second and third lead screws.
8. The endoscopic stitching device according to paragraph 2, wherein the handle assembly further includes a second actuation switch configured to actuate the second and third motors in order to cause reciprocating axial displacement of the first and second blade drive members in opposite directions.
9. The endoscopic stitching device according to paragraph 1, wherein the handle assembly further includes a battery pack electrically coupled to the actuation assembly and the processor to supply power thereto.
10. The endoscopic stitching device according to paragraph 9, wherein the batter pack is removably attached to a housing of the handle assembly.
11. The endoscopic stitching device according to paragraph 1, wherein the handle assembly further includes a third actuation switch operatively coupled with the processor and the second and third motors such that actuation of the third actuation switch causes retraction of the first and second blades.
12. The endoscopic stitching device according to paragraph 11, wherein actuation of the third actuation switch causes axial displacement of the first and second blades in a proximal direction.
13. The endoscopic stitching device according to paragraph 1, wherein proximal displacement of the main rod transitions the first and second jaws to the closed position.
14. A powered handle assembly for use with a stitching device comprising:
   an actuation assembly including first, second, and third motors;
   a processor electrically connected to the first, second, and third motors to control actuation of the first, second, and third motors;
   a first actuation switch electrically connected to the processor to control actuation of the first motor operatively coupled with a main rod of the stitching device; and
   a second actuation switch electrically connected to the processor to control actuation of the second and third motors coupled with respective first and second blade drive members of the stitching device, wherein actuation of the first actuation switch causes axial displacement of the main rod of the stitching device, and actuation of the second actuation switch causes actuation of the second and third motors, which, in turn, causes axial displacement of the first and second blade drive members in opposite directions.
15. The powered handle assembly according to paragraph 14, wherein the actuation of the second actuation switch, while the first actuation switch is actuated, causes actuation of the second and third motors.
16. The powered handle assembly according to paragraph 14, further comprising a third actuation switch configured to cause actuation of the second and third motors such that the first and second blade drive members are retracted.
17. The powered handle assembly according to paragraph 16, wherein actuation of the third actuation switch causes axial displacement of the first and second blade drive members in a proximal direction.
18. The powered handle assembly according to paragraph 14, wherein the actuation of the first actuation switch causes axial displacement of the main rod of the stitching device in a proximal direction.
19. The powered handle assembly according to paragraph 14, further comprising a battery pack electrically coupled to the processor and the actuation assembly to supply power thereto.
20. The powered handle assembly according to paragraph 19, wherein the battery pack is detachably coupled to a housing of the handle assembly

## Claims

1. An endoscopic stitching device comprising:
a handle assembly including:
an actuation assembly including first, second, and third motors; and
a processor electrically connected to the first, second, and third motors, the processor configured to control actuation of the first, second, and third motors; and
an elongate shaft assembly including:
a main rod operatively coupled with the first motor of the actuation assembly such that actuation of the first motor causes axial displacement of the main rod;
first and second blade drive members operatively coupled with the second and third motors, respectively, such that actuation of the second and third motors causes axial displacement of the first and second blade drive members; and
a tool assembly including:
first and second jaws operatively coupled with the main rod of the elongate shaft assembly such that axial displacement of the main rod transitions the first and second jaws between open and closed positions; and
first and second blades slidably disposed in the respective first and second jaws, each of the first and second blades configured to engage a needle received in the first or second jaws, the first and second blades operatively coupled with the first and second blade drive members, respectively, such that actuation of the second and third motors causes axial displacement of the first and second blades.

2. The endoscopic stitching device according to claim 1, wherein the handle assembly further includes a first actuation switch configured to actuate the first motor to impart axial displacement to the main rod, which in turn, transitions the first and second jaws between the open and closed positions.

3. The endoscopic stitching device according to claim 2, wherein the handle assembly further includes a potentiometer operatively coupled to the first actuation switch to enable proportional control of the jaws; preferably wherein the first actuation switch further includes a gear assembly operatively coupled with the potentiometer to improve sensor resolution.

4. The endoscopic stitching device according to any preceding claim, wherein the handle assembly further includes a first lead screw coupled with an output shaft of the first motor for concomitant rotation therewith, and a first coupling nut threadably coupled with the first lead screw and securely fixed with the main rod such that actuation of the first motor causes axial displacement of the main rod.

5. The endoscopic stitching device according to any preceding claim, wherein the handle assembly further includes second and third lead screws coupled with respective output shafts of the second and third motors for concomitant rotation therewith, and second and third coupling nuts threadably coupled with the respective second and third lead screws and securely fixed with the respective first and second blade drive members such that actuation of the second and third motors causes axial displacement of the respective first and second blades; preferably wherein the handle assembly further includes second and third guide blocks configured to slidably receive a least a portion of the respective second and third coupling nuts thereon, while inhibiting rotation of the second and third coupling nuts about the respective second and third lead screws.

6. The endoscopic stitching device according to claim 2, wherein the handle assembly further includes a second actuation switch configured to actuate the second and third motors in order to cause reciprocating axial displacement of the first and second blade drive members in opposite directions.

7. The endoscopic stitching device according to claim 1, wherein the handle assembly further includes a battery pack electrically coupled to the actuation assembly and the processor to supply power thereto; preferably wherein the batter pack is removably attached to a housing of the handle assembly.

8. The endoscopic stitching device according to any preceding claim, wherein the handle assembly further includes a third actuation switch operatively coupled with the processor and the second and third motors such that actuation of the third actuation switch causes retraction of the first and second blades; preferably wherein actuation of the third actuation switch causes axial displacement of the first and second blades in a proximal direction.

9. The endoscopic stitching device according to any preceding claim, wherein proximal displacement of the main rod transitions the first and second jaws to the closed position.

10. A powered handle assembly for use with a stitching device comprising:
an actuation assembly including first, second, and third motors;
a processor electrically connected to the first, second, and third motors to control actuation of the first, second, and third motors;
a first actuation switch electrically connected to the processor to control actuation of the first motor operatively coupled with a main rod of the stitching device; and
a second actuation switch electrically connected to the processor to control actuation of the second and third motors coupled with respective first and second blade drive members of the stitching device, wherein actuation of the first actuation switch causes axial displacement of the main rod of the stitching device, and actuation of the second actuation switch causes actuation of the second and third motors, which, in turn, causes axial displacement of the first and second blade drive members in opposite directions.

11. The powered handle assembly according to claim 10, wherein the actuation of the second actuation switch, while the first actuation switch is actuated, causes actuation of the second and third motors.

12. The powered handle assembly according to claim 11, further comprising a third actuation switch configured to cause actuation of the second and third motors such that the first and second blade drive members are retracted; preferably wherein actuation of the third actuation switch causes axial displacement of the first and second blade drive members in a proximal direction.

13. The powered handle assembly according to any of claims 10 to 12, wherein the actuation of the first actuation switch causes axial displacement of the main rod of the stitching device in a proximal direction.

14. The powered handle assembly according to any of claims 10 to 13, further comprising a battery pack electrically coupled to the processor and the actuation assembly to supply power thereto.

15. The powered handle assembly according to claim 14, wherein the battery pack is detachably coupled to a housing of the handle assembly
